# EUROPEAN PATENT APPLICATION

(11) **EP 4 516 927 A1**
(43) Date of publication of application: **05.03.2025**
(21) Application number: 23194414.1
(22) Date of filing: 31.08.2023
(51) Int. Cl.: C12Q 1/6883

(54) **GALECTIN-3, A BIOMARKER OF LYSOSOMAL DAMAGE IN MUSCULAR DYSTROPHY**

(71) Applicant: GENETHON, 91000 Evry-Courcouronnes (FR); Institut National de la Santé et de la Recherche Médicale, 75013 Paris (FR); Université d'Evry Val d' Essonne, 91000 Evry-Courcouronnes (FR)
(72) Inventor: Israeli, David, 75012 Paris (FR); Jaber, Abbass, 94700 Maisons-Alfort (FR)
(74) Representative: Axe PI

(57) **Abstract**

The present invention relates to the use of galectin-3 or galectin-3 mRNA as a biomarker for the diagnosis, the prognosis, for monitoring the evolution, or for determining the risk of having or of developing a muscular dystrophy, or for determining the efficacy of a treatment of a muscular dystrophy.

## Description

### FIELD OF THE INVENTION

The present invention relates to the use of galectin-3 or galectin-3 mRNA as a lysosomal damage biomarker in muscular dystrophy. It also relates to a method for the diagnosis, the prognosis, for monitoring the evolution, or for determining the risk of having or for developing a muscular dystrophy in a subject. It also relates to a method for determining the efficacy of a treatment of a muscular dystrophy in a subject.

### BACKGROUND

Muscular dystrophies (MDs) are a group of genetic diseases. The group is characterized by progressive weakness and degeneration of the skeletal muscles that control movement. Some forms of MDs develop in infancy or childhood, while others may not appear until middle age or later. The disorders differ in terms of the distribution and extent of muscle weakness (some forms of MDs also affect cardiac muscle), the age of onset, the rate of progression, and the pattern of inheritance.

Duchenne muscular dystrophy (DMD) is the most common form of muscular dystrophy. Genetic and biochemical research over the years has characterized the cause, pathophysiology and development of the disease providing several potential therapeutic targets.

DMD is a severe muscle-wasting disease caused by genetic mutations in the DMD gene encoding a structural protein called dystrophin. Mutations in the same gene are responsible for the milder form of the disease, which is called Becker muscular dystrophy (BMD). Protein truncating mutations cause Duchenne form of the disease characterized by complete or almost complete absence of dystrophin, while BMD patients have in frame mutations and are partly protected from muscular degeneration by reduced levels of (smaller or semi-functional) dystrophins. (Aartsma-Rus A, Spitali P. Circulating Biomarkers for Duchenne Muscular Dystrophy. J Neuromuscul Dis. 2015 Jul 22;2(s2):S49-S58. doi: 10.3233/JND-150102. PMID: 27858763; PMClD: PMC5271432.)

Another form of MDs is limb girdle muscular dystrophies (LGMD). LGMDs are rare conditions, and they present differently in different people with respect to age of onset, areas of muscle weakness, heart and respiratory involvement, rate of progression and severity. LGMDs can begin in childhood, adolescence, young adulthood or even later. Both genders are affected equally. LGMDs cause weakness in the shoulder and pelvic girdle, with nearby muscles in the upper legs and arms sometimes also weakening with time. Weakness of the legs often appears before that of the arms. Facial muscles are usually unaffected. As the condition progresses, people can have problems with walking and may need to use a wheelchair over time. The involvement of shoulder and arm muscles can lead to difficulty in raising arms over head and in lifting objects. Physical therapy is often recommended to retain muscle strength and mobility for as long as possible. Stretching, mechanical aids, or surgery may also aid in that goal. In some types of LGMD, the heart and breathing muscles may be involved. As muscles deteriorate, a ventilator may be required to help with breathing. Cardiac surveillance is recommended, and those who develop heart problems should consult a cardiologist for appropriate treatment.

LGMD is a heterogeneous group of genetic forms of muscular dystrophy with many subtypes that are each caused by a unique mutation, generating compilation of symptoms.

There are several forms of LGMD, and the forms are classified by their associated genetic defects.

Many biomarkers have been described for muscular dystrophy. For example, muscle creatin kinase (mCK) is a widely used biomarker for myofiber degeneration (Ozawa E, Hagiwara Y, Yoshida M. Creatine kinase, cell membrane and Duchenne muscular dystrophy. Mol Cell Biochem. 1999 Jan;190(1-2):143-51. PMID: 10098981.). However, mCK has the major disadvantages of not being specific, to be highly variable between patients, and to have a relatively high rate of false positive. Indeed, serum creatine kinase levels can vary from day to day in the same patient, making them unreliable indicators of muscle changes under a therapy. In particular, serum creatine kinase would correlate to the degradation of muscle tissue under physical efforts rather than to the pathological state of the patient. For example, it has been shown that the level of this marker decreases as soon as the patient is in a wheelchair.

Myomesin-3 described in WO2015/104403 patent application, is also a biomarker used for muscular dystrophy.

WO2014/049282 patent application also describes a method that is used for muscular dystrophy diagnosis, prognosis and therapeutic monitoring, by detecting titin or one or more fragments of titin in a bodily fluid of a patient.

Currently, despite the availability of these methods, there is still an urgent need for the development of more analytical tools for the diagnosis, prognosis, monitoring or evaluation of the efficacy of treatments for such conditions.

Indeed, the expression of microdystrophin, the transgene itself, was used as a biomarker for outcome evaluation in Sarepta Therapeutics' gene transfer clinical trials for DMD. This was approved by the FDA because of the lack of other clinical outcome biomarkers (Chamberlain, Jeffrey S et al. "Microdystrophin Expression as a Surrogate Endpoint for Duchenne Muscular Dystrophy Clinical Trials." Human gene therapy vol. 34,9-10 (2023): 404-415. doi:10.1089/hum.2022.190).

Hence, there remains a need for a readily specific biomarker reflecting the progression of muscular dystrophies.

### PROBLEM TO BE SOLVED

The technical problem underlying the present invention is notably to provide a specific biomarker reflecting the progression of muscular dystrophies.

The technical problem is solved by the embodiments provided herein and as characterized in the appended claims.

### SUMMARY OF THE INVENTION

In a first aspect, the invention relates to the use of galectin-3 or galectin-3 mRNA as a biomarker for the diagnosis, the prognosis, for monitoring the evolution, or for determining the risk of having or of developing a muscular dystrophy, or for determining the efficacy of a treatment of a muscular dystrophy.

In another aspect, the invention relates to a method for the diagnosis, the prognosis, for monitoring the evolution, or for determining the risk of having or for developing a muscular dystrophy in a subject, or for determining the efficacy of a treatment of a muscular dystrophy in a subject, comprising detecting the presence or absence of galectin-3 or galectin-3 mRNA upregulation.

In a further aspect, the invention relates to the use of a kit for implementing a method according to the invention, comprising an analytical test to identify and quantify the presence or absence of galectin-3 upregulation for the quantification of lysosome damage in muscular dystrophy.

The Applicant has been able to demonstrate that the galectin-3 is upregulated in muscular dystrophy.

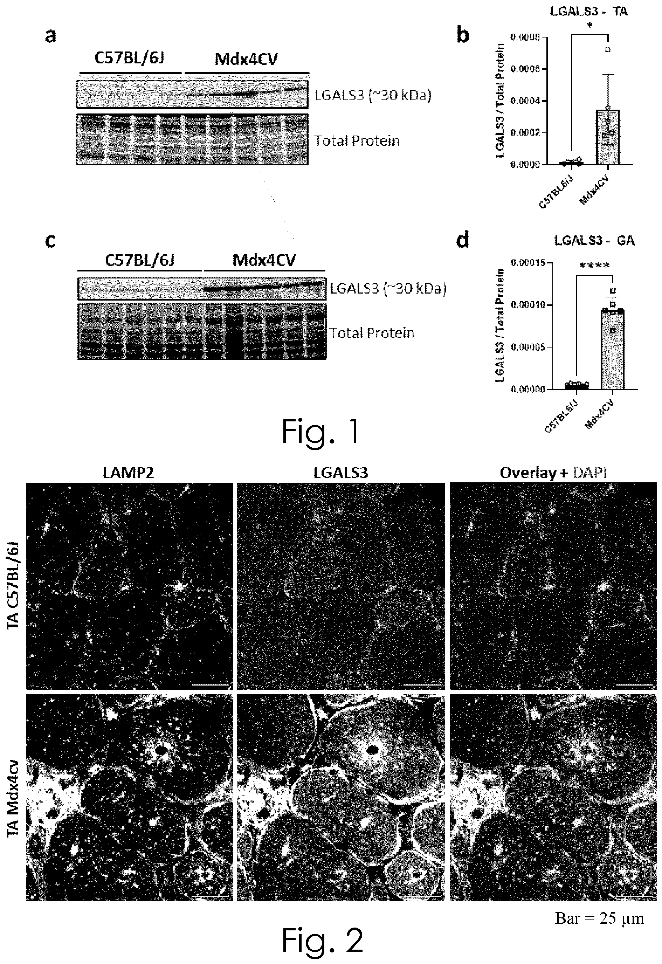

Further aspects and advantages of the present invention are described in the following description (with reference to Figures 1 to 8), which should be regarded as illustrative and not limiting the scope of the present application.

### BRIEF DESCRIPTION OF THE DRAWINGS

**Figure 1** shows Gal-3 expression in normal (C57BI6/J) and dystrophic (MDX4CV) muscle. **(1a-b)** *Tibialis Anterior* (TA) muscle of 7 weeks old mdx mice and its quantification (N=4 or 5). **(1c-d)** Gastrocnemius (GA) muscle of the same mice (^{∗} p≤ 0.05; ^{∗∗∗∗} p≤ 0.001).
**Figure 2** illustrates immunodetection of Lamp-2 and galectin-3 in normal and dystrophic GA muscle. In the left column of figure 2, Lamp2 that stains specifically the lysosome, is increasingly expressed in the TA muscle of the mdx mouse compared to the WT C57BI/6 mouse, which indicates lysosomal perturbation and enlargement in the mdx muscle. In the middle column, the "Puncta" pattern of LGALS3 stain, is increasingly visible in the mdx muscle compared to the muscle of the healthy control mouse. In the right column, colocalization of LGALS3 (red) and the lysosomal marker LAMP2 (green) is visible in the mdx muscle.

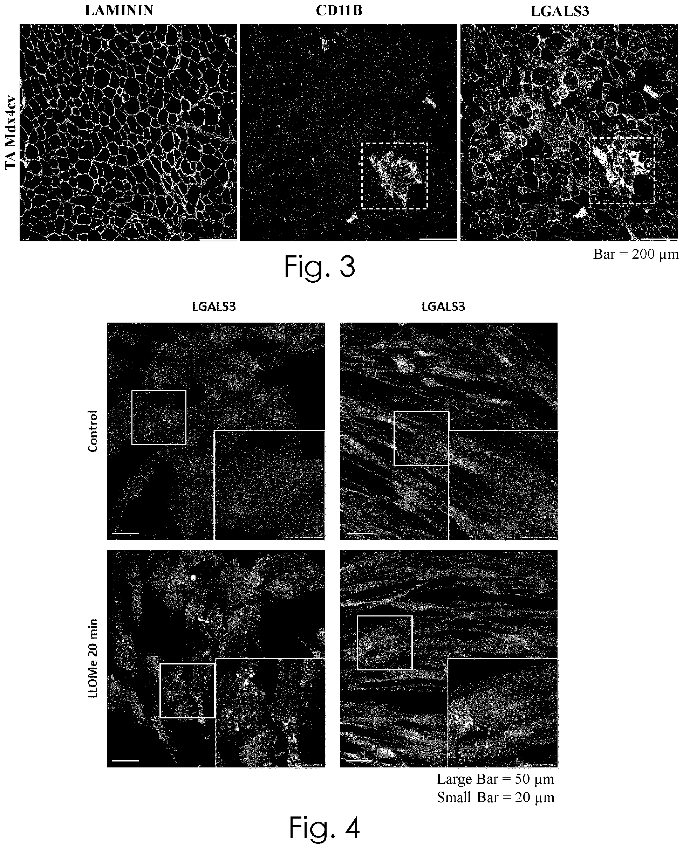

**Figure 3** shows Gal-3 expression in the dystrophic muscle on both CD11b invading immunoinflammatory cells (square dotted lines), and inside (CD11b negative) myofibers. On the left, laminin stain detects muscle myofibers. In the middle, invading inflammatory cells are detected by the anti-CD11b antibody. On the right, the anti-Gal-3 antibody detects Gal-3 expression on both myofibers and the invading inflammatory (CD11b positive) cells.
**Figure 4** shows Gal-3 puncta assay for the detection of lysosome damage in pure myogenic lineage. The left side of this panel presents cultured human myoblasts. The right side of the panel presents the myotubes that were derived from the myoblasts, which represents an *in vitro* model for myogenic differentiation. On the bottom, the myoblasts (bottom left) and myotubes (bottom right) are treated by the lysosomal damaging substance LLOME. The pattern of Galectin-3, which is cytoplasmic diffused in untreated cultures, become lysosomal "punctuated" after LLOME treatment.

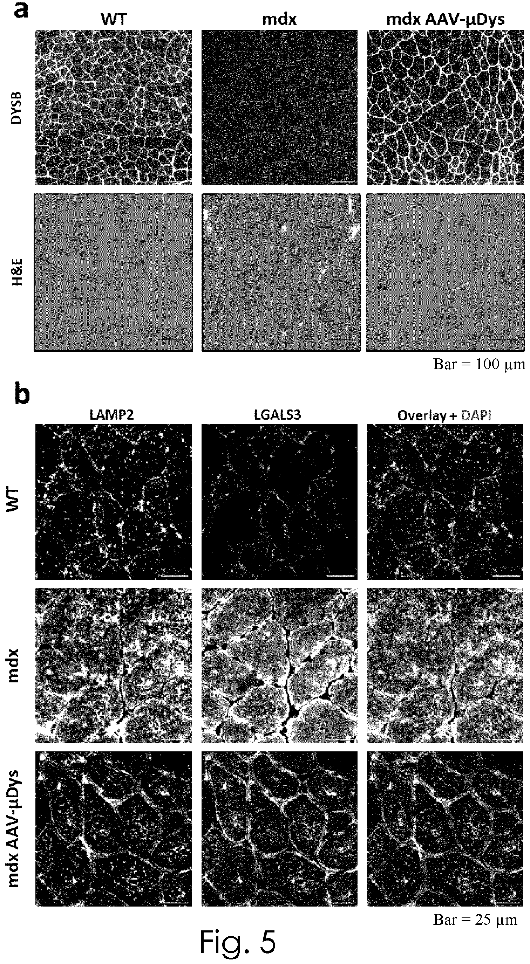

**Figure 5** illustrates the monitoring of lysosomal damage in gene therapy treated mdx model mice (labeled mdx AAV-µdys) compared to mdx mice and control wild-type (WT) mice. In the upper panel (a), dystrophin expression (top line) in the TA muscle has been detected, 1 month after gene therapy treatment. Note in particular, on the upper right panel, that 100% of the fibers of the treated mouse are dystrophin positive. In the H&E stain (bottom line), the normal architecture of the tissue is restored in the treated mouse (on the right). This data confirms the correct expression and function of the µdystrophin transgene.
   In the bottom panel: Immunodetection of the lysosomal-damage biomarker Gal-3 and of the lysosomal marker Lamp2, in WT mouse (Up), mdx mouse (middle), and Gene-therapy treated mdx mouse (bottom) is visible. Increased expression of Lamp2 and Lgals-3 and their colocalization (Lamp2 green, Gal-3 red) is visible in the mdx (middle line) compared to the WT, while as the gene-therapy treated mice (bottom line) present reduced Gal-3 staining, which indicates reduced lysosomal damage.

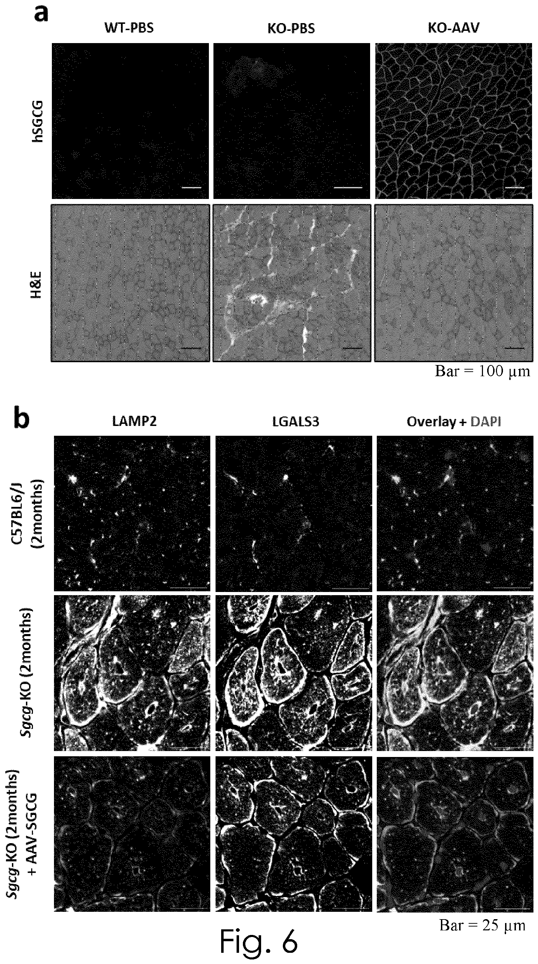

**Figure 6** illustrates the monitoring of lysosomal damage in gene therapy treated mouse model for g- sarcoglycanopathy (labeled KO-AAV) compared to a knock-out mice (KO) and control wild-type (WT) mice. Upper Panel (a) top line: Immunodetection of SGCG expression in the TA muscle 1 month after gene-therapy treatment. The AAV-γ-sarcoglycan vector codes for the human form of gammasarcoglycan (PMID: 31194043). The antibody for the detection of the Gammasarcoglycan protein is human-specific (i.e. cannot detect the mouse gammasarcoglycan in the normal mouse) and therefore both the healthy (WT) and the null (KO-PBS) mice are negative. In contrast, the gene therapy treated KO-mouse (KO-AAV upper panel right figure 6) present nearly complete restoration of gammasarcoglycan expression. In the H&E staining (bottom line), the normal architecture of the tissue is restored in the treated mouse on the right. This data indicates the correct expression and function of the human γ-sarcoglycan transgene.
   In the bottom panel (b), immunodetection of the lysosomal-damage biomarker Gal-3 and of the lysosomal marker Lamp2 is observed. The expression of both Lamp2 and Gal-3 and their colocalization (Lamp2 green, Gal-3 red), which is upregulated in the muscle of the knockout mouse (middle line), is normalized after gene-therapy treatment for the restoration of g-sarcoglycan expression (bottom line.

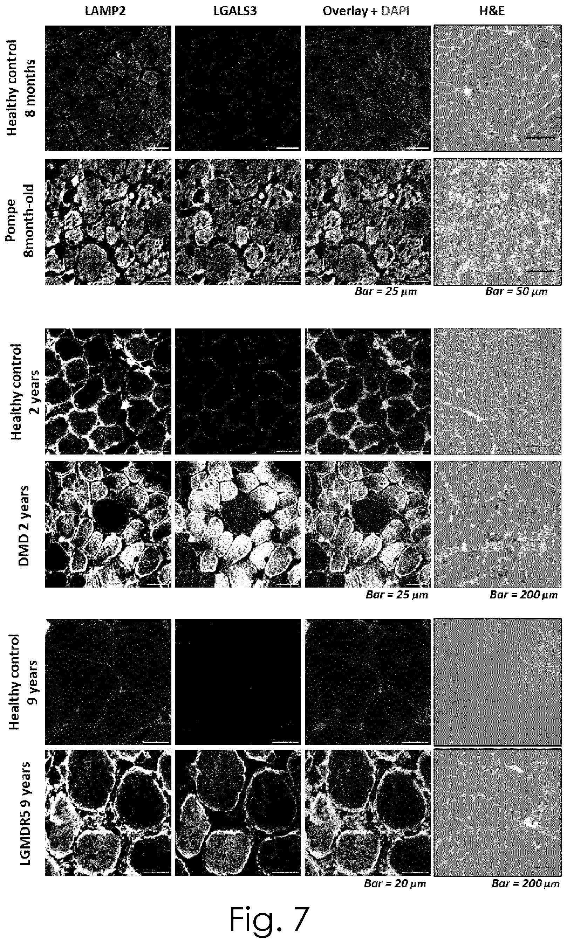

**Figure 7** illustrates the detection of lysosomal damage in human muscular dystrophy. (Upper panel) skeletal muscle transversal sections of an eight months old GSD2 (Pompe) patient. (middle panel) transversal sections of 2-years old Duchenne patient. (lower panel) transversal sections of a nine-year old SGCG (LGMD R5, also named gamma sarcoglycanopathy) human patient. For all panels: Immunodetection of the lysosomal marker LAMP2 (1^{st} column), lysosomal-damage biomarker Gal-3 (2^{nd} column) and their colocalization (3^{rd} column) as well as H&S stain (4^{th} column) for human patients (bottom line of each panel) vs age-matched healthy controls (top line of each panel).

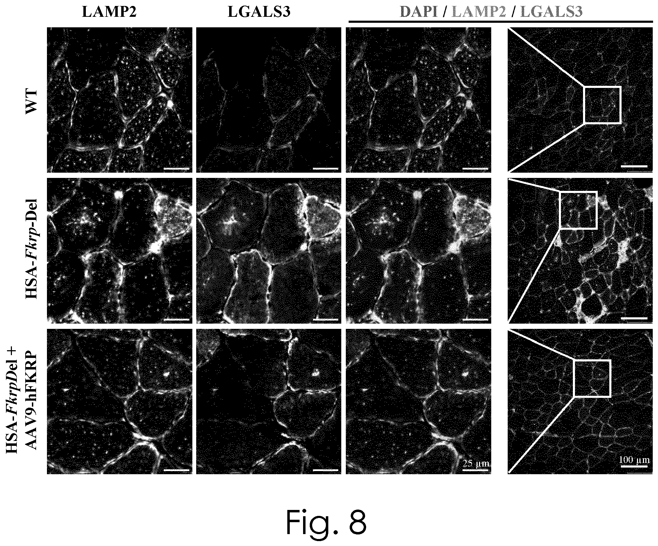

**Figure 8** illustrates the detection of lysosomal damage in gene therapy treated mouse model for the FKRP related limb girdle muscular dystrophy R9 (LGMDR9), (labeled HSA-Fkrp-Del AAV9 hFKRP) compared to a knock-out mice (labeled HSA-Fkrp-Del) and control wild-type (WT) mice. At the age of 1 month, the mice model for LGMDR9 (FKRP-null) were gene-therapy treated for the restoration of FKRP expression. Three months later, at the age of 4 month the mice were sacrificed, the correct FKRP expression in the muscles was detected (not shown), and transversal sections of the TA muscle were analyzed. Immunodetection of the lysosomal-damage biomarker Gal-3 and of the lysosomal marker Lamp2, in WT mouse (Up), FKRP-null mouse (middle), and Gene-therapy treated FKRP-null mouse (bottom) is visible. Increased expression of Lamp2 and Lgals-3 and their colocalization (Lamp2 green, Gal-3 red) is visible in the FKRP-null mouse (middle line) compared to the WT, while as the gene-therapy treated mice (bottom line) present reduced Gal-3 staining, which indicates reduced lysosomal damage.

### DETAILED DESCRIPTION

The present invention provides a novel biomarker for diagnosis muscular dystrophies as defined in the appended claims. The level of the biomarker in a biological fluid, or in muscles, in particular in muscle samples extracted from *tibialis anterior,* gastrocnemius, the biceps, the deltoid, or the quadriceps, according to the present invention is low in normal state and upregulated in the case of muscular dystrophies. Accordingly, the marker is useful for diagnosis intended to determine whether or not a patient carries muscular dystrophy, prediction of the development of muscular dystrophy, screening for a therapeutic agent or technique for muscular dystrophy treatment, evaluation of the efficacy of a therapeutic agent or technique for muscular dystrophy treatment, and other purposes. Such a biomarker can also be used for detecting and monitoring muscle lysosomal damage in diseases or disorders which are not primary muscle disease but involving muscle damage, such as neuromuscular, neurodegenerative, cardiovascular, metabolic, and lysosomal storage diseases.

The term "biological fluid" as used herein preferably refers to blood, serum, plasma, saliva and urine. According to a particular embodiment of the invention, the biological fluid sample is a serum or plasma sample.

The term "therapeutic agent" as used herein refers to a drug, molecule, nucleic acid, protein, composition, or other substance that provides a therapeutic effect. The terms "therapeutic agent" and "active agent" are used interchangeably. In a particular embodiment, the therapeutic agent is a gene therapy agent. In a more particular embodiment, the gene therapy agent is an AAV-based gene therapy product or a lentiviral-based gene therapy product. In an advantageous embodiment, the gene therapy agent is an AAV-based gene therapy product. In a more advantageous embodiment, the AAV-based gene therapy product comprises a transgene encoding a dystrophin or microdystrophin protein, an alpha-sarcoglycan protein, a beta-sarcoglycan protein, a gamma-sarcoglycan protein, a delta-sarcoglycan protein, a fukutin-related protein (FKRP), a dynamin-2 protein or a calpain-3 protein, a dysferlin or mini-dysferlin protein, a myotubularin (MTM1) protein and a spinal muscular atrophy (SMA) protein. In an even more advantageous embodiment, said AAV-based gene therapy product comprises a transgene encoding a dystrophin or microdystrophin protein, an alpha-sarcoglycan protein, a beta-sarcoglycan protein, a gamma-sarcoglycan protein, a delta-sarcoglycan protein, a fukutin-related protein (FKRP), a dynamin-2 protein or a calpain-3 protein, a dysferlin or mini-dysferlin protein. In an even more advantageous embodiment, said AAV-based gene therapy product comprises a transgene encoding a dystrophin or microdystrophin protein, a gamma-sarcoglycan protein, a fukutin-related protein (FKRP), or a dynamin-2 protein.

The invention relates to the use of galectin-3 or galectin-3 mRNA as a biomarker for the diagnosis, the prognosis, for monitoring the evolution, or for determining the risk of having or of developing, or for determining the efficacy of a treatment of a metabolic disorder having muscular damage including diabetes, cachexia and metabolic syndrome; a neuromuscular disorder such as amyotrophic lateral sclerosis (ALS), spinal muscular atrophy (SMA) and muscular dystrophy; and/or Pompe disease.

The invention relates, in a first aspect, to the use of galectin-3 or galectin-3 mRNA as a biomarker for the diagnosis, the prognosis, for monitoring the evolution, or for determining the risk of having or of developing a muscular dystrophy and/or Pompe disease.

Advantageously, the invention relates to the use of galectin-3 or galectin-3 mRNA as a biomarker for the diagnosis, the prognosis, for monitoring the evolution, or for determining the risk of having or of developing a muscular dystrophy.

The term "diagnosis" as used herein refers to the identification of the nature of a disease or medical condition of a subject that may experience symptoms. The information required for diagnosis is typically collected from a history and physical examination of such subject and can involve additional medical tests. Muscular dystrophy (MD) diagnosis usually involve multiple tests for a complete assessment including tests to evaluate muscle functions (heart-monitoring tests such as electrocardiography and echocardiogram, lung-monitoring tests and/or electromyography), muscle biopsies, enzyme tests (such as creatine phosphokinase (CpK3)), and/or genetic testing.

In the present invention, the method of diagnosis is preferably a muscle biopsy immunoassays and/or genetic testing, more preferably muscle biopsy.

The term "genetic testing" as used herein refers to a test to reveal mutations in genes that may cause illness or disease. Three major types of genetic testing are available in laboratories: cytogenetic to examine whole chromosomes, biochemical to measure protein produced by genes, and molecular to look for small DNA mutations.

The term "muscle biopsy" as used herein refers to the removal of a small piece of muscle tissue for examination. There are two types of muscle biopsy: needle biopsy and open biopsy. A needle biopsy involves inserting a needle into the muscle. When the needle is removed, a small piece of tissue remains in the needle. More than one needle stick may be needed to get a large enough sample. An open biopsy involves making a small cut in the skin and into the muscle. The muscle tissue is then removed. Analysis of the muscle tissue can distinguish muscular dystrophies from other muscle diseases.

The term "determining the risk" as used herein refers to any kind of procedure aiming to obtain information instrumental in the assessment whether a patient is likely or more likely than the average or a comparative subject, the latter preferably having similar symptoms, to suffer from a certain disease or disorder in the past, at the time of the diagnosis or in the future, even before the apparition of symptoms.

The term "prognosis" as used herein refers to a prediction of the probable course and outcome of a clinical condition or disease. A prognosis is usually made by evaluating factors or symptoms of a disease that are indicative of a favorable or unfavorable course or outcome of the disease.

The term "monitoring" as used herein refers to the use of biomarkers to regularly screen the patients for a change in the degree or extent of disease, e.g. MD evolution.

The invention also relates to the use of galectin -3 or galectin-3 mRNA as a biomarker in screening for a therapeutic agent.

The term 'screening for a therapeutic agent" as used herein refers to a method of investigation for screening natural or synthetic substances with unknown pharmacological properties, with a view to drug discovery. Only molecules showing interesting activity are pre-selected, in systematic trials on one or more tests, depending on the program adopted.

Galectin-3 or galectin-3 mRNA biomarkers according to the invention, can further be used for determining the efficacy of a treatment of a muscular dystrophy.

In an alternative embodiment, galectin-3 or galectin-3 mRNA biomarkers according to the invention, can be used for the diagnosis, the prognosis, for monitoring the evolution, or for determining the risk of having or of developing Pompe disease, or for determining the efficacy of a treatment of Pompe disease.

The term "efficacy of a treatment" as used herein refers to the degree to which a treatment accomplishes the desired or projected outcomes, for instance the ability of a therapeutic agent to achieve the desired effect.

The term "treatment" as used herein refers to the alleviation of symptoms of a particular disorder in a patient, or the improvement of an ascertainable measurement associated with a particular disorder. Treatment also includes prophylaxis which refers to preventing a disease or condition or preventing the occurrence of symptoms of such a disease or condition, in a patient. As used herein, the term "patient" refers to a mammal, including a human, in particular a human, more particularly a child or a teenager.

Galectin-3 (*in homo sapiens, UniprotKB* / *Swiss-Prot accession number P17931*), also called as LGALS3, CBP35, GAL3, GALBP, GALIG, L31, LGALS2, MAC2, lectin, galactoside binding soluble 3, is a polypeptide that in humans is encoded by the LGALS3 gene. Galectin-3 is a member of the lectin family. The molecular weight of said galectin-3 is approximately 30 kDa and, like all galectins, contains a carbohydrate-recognition-binding domain (CRD) of about 130 amino acids that enable the specific binding of β-galactosides. Indeed, it has an affinity for beta-galactosides and exhibits antimicrobial activity against bacteria and fungi. The said polypeptide has been shown to be involved in the following biological processes: cell adhesion, cell activation and chemoattraction, cell growth and differentiation, cell cycle, apoptosis, lysosomal repair, degradation and replacement.

The term "polypeptide(s)" as used herein refers to any peptide or protein comprising two or more amino acids joined to each other by peptide bonds or modified peptide bonds. "Polypeptide(s)" refers to both short chains, commonly referred to as peptides, oligopeptides, and oligomers and to longer chains generally referred to as proteins. Polypeptides can contain amino acids other than the 20 gene encoded amino acids. "Polypeptide(s)" include those modified either by natural processes, such as processing and other post-translational modifications, but also by chemical modification techniques. Such modifications are well described in research literature, and are well known to those skilled in the art.

The terms "muscular dystrophy" or "muscular dystrophies" or "MD" as used herein refer to a group of rare genetic neuromuscular diseases that gradually cause the skeletal muscles to weaken and break, leading to an increasing level of disability. Over time, muscle weakness decreases mobility, making everyday tasks difficult. Although MD can affect several body tissues and organs, it most prominently affects the integrity of muscle fibers. Also, overall muscle strength and tendon reflexes are usually lessened or lost due to replacement of muscle by connective tissue and fat. There are many kinds of muscular dystrophy, each affecting specific muscle groups, with signs and symptoms appearing at different ages, and varying in severity. MD is caused by changes (mutations) in one of the genes responsible for the structure and functioning of a person's muscles. The mutations are often inherited from a person's parents but can also occur spontaneously. They cause changes in the muscle fibers that interfere with the muscles' ability to function. Muscular dystrophies may be X-linked recessive, autosomal recessive, or autosomal dominant.

In one embodiment, MD denote Duchenne muscular dystrophy (DMD), Centronuclear myopathy related to dynamin 2 mutations, myotonic dystrophy, facioscapulohumeral muscular dystrophy, Becker muscular dystrophy (BMD), distal muscular dystrophy, congenital muscular dystrophy, Emery-Dreifuss muscular dystrophy, oculopharyngeal muscular dystrophy, limb-girdle muscular dystrophies (LGMD), or Miyoshi muscular dystrophy.

In a preferred embodiment, MD denote Duchenne muscular dystrophy (DMD), LGMD, or Centronuclear myopathy related to dynamin 2 mutations.

Limb-girdle muscular dystrophies (LGMD) refer to a group of MDs that affect mostly proximal skeletal muscles (hip and shoulder muscles) leading to progressive, predominantly proximal muscle weakness of the muscles on or close to the torso caused by a loss of muscle fibres. Usually, the hip girdle is the first area to exhibit weakness, manifesting as difficulty walking, going up and/or downstairs, rising from a chair, bending at the waist, or squatting. Weakness of the shoulder girdle can make lifting objects, or even elevating the arms, difficult or impossible. Rate of progression varies between patients. The disease commonly leads to dependence on a wheelchair within years of symptom onset, although some patients maintain mobility Eventually the disease can affect other muscles such as the ones located in the face. LGMDs are classified in subtypes and caused by a mutation in a gene encoding a protein, which is specific to each subtype. LGMD inheritance is autosomal. In the current classification, each subtype name is followed by either R or D to indicate recessive or dominant inheritance, as well as by a number determined by the order of discovery.

In a particular embodiment, LGMD refers to alpha-sarcoglycanopathy (LGMD R3), gamma-sarcoglycanopathy (LGMD R5), dysferlinopathy (LGMD R2), FKRP related limb girdle muscular dystrophy (LGMD R9).or calpainopathy (LGMD R1). In another particular embodiment, LGMD also include all conditions described in Table 1 of Bouchard and Tremblay, J. Clin. Med 2023, 12,4769 hereby enclosed herein. In a preferred embodiment, LGMD is gamma-sarcoglycanopathy (LGMD R5), or FKRP related limb girdle muscular dystrophy (LGMD R9).

In an alternative embodiment, galectin-3 or galectin-3 mRNA according to the present invention is also used for the diagnosis, the prognosis, for monitoring the evolution, or for determining the risk of having or of developing amyotrophic lateral sclerosis (ALS), spinal muscular atrophy (SMA), metabolic diseases having muscular damage including diabetes, cachexia and metabolic syndrome, or for determining the efficacy of a treatment of ALS, SMA, metabolic diseases having muscular damage including diabetes, cachexia and metabolic syndrome.

Duchenne Muscular Dystrophy (DMD) is an X-linked recessive disorder. DMD symptom onset is in early childhood, usually between ages 2 and 3. The disease primarily affects boys, but in rare cases it can affect girls. It is characterized by muscle fiber wasting, functional impairment and eventual death due to respiratory and heart failure. The underlying causes are frame-shifting and nonsense mutations in the DMD gene, resulting in the absence of functional dystrophin protein. The muscle protein, dystrophin, is in most muscle cells. Intact dystrophin anchors the intracellular cytoskeleton to the extracellular matrix, works to strengthen the muscle fibers and thereby prevents membrane damage as muscles contract and relax.

Myotonic dystrophy is a type of muscular dystrophy. In myotonic dystrophy, muscles are often unable to relax after contraction. Other manifestations may include cataracts, intellectual disability, and heart conduction problems. In men, there may be early balding and an inability to father children. While myotonic dystrophy can occur at any age, onset is typically in the 20s and 30s. Myotonic dystrophy is caused by a genetic mutation in one of two genes. Mutation of the DMPK gene causes myotonic dystrophy type 1 (DM1). Mutation of CNBP gene causes type 2 (DM2). Myotonic dystrophy is typically inherited, following an autosomal dominant inheritance pattern, and it generally worsens with each generation. A type of DM1 may be apparent at birth. DM2 is generally milder.

Facioscapulohumeral muscular dystrophy (FSHD) is a type of muscular dystrophy that tends to sequentially weaken the muscles of the face, those that position the scapula, and those overlying the humerus bone of the upper arm. These areas can be spared, and muscles of other areas usually are affected, especially those of the chest, spine, abdomen, and shin. Almost any skeletal muscle can be affected in severe disease. Abnormally positioned, or winged, scapulas are common, as is the inability to lift the foot, known as foot drop. The two sides of the body are often affected unequally. Weakness typically manifests at ages 15 - 30 years. FSHD can also cause hearing loss and blood vessel abnormalities in the back of the eye. FSHD is caused by a genetic mutation leading to deregulation of the DUX4 gene. Normally, DUX4 is expressed (i.e., turned on) in cells of the ovary and in very early human development, becoming repressed (i.e., turned off) by the time an embryo is several days old. In FSHD, DUX4 is inadequately repressed, allowing sporadic expression throughout life. Deletion of DNA in the region surrounding DUX4 is the causative mutation in 95% of cases, termed "D4Z4 contraction" and defining FSHD type 1 (FSHD1). FSHD caused by other mutations is FSHD type 2 (FSHD2). For disease to develop, also required is a 4qA allele, which is a common variation in the DNA next to DUX4. The chances of a D4Z4 contraction with a 4qA allele being passed on to a child is 50% (autosomal dominant); in 30% of cases, the mutation arose spontaneously. Mutations of FSHD cause inadequate DUX4 repression by unpacking the DNA around DUX4, making it accessible to be copied into messenger RNA (mRNA). The 4qA allele stabilizes this DUX4 mRNA, allowing it to be used for production of DUX4 protein. DUX4 protein is a modulator of hundreds of other genes, many of which are involved in muscle function.

Becker muscular dystrophy is an X-linked recessive inherited disorder characterized by slowly progressing muscle weakness of the legs and pelvis. It is a type of dystrophinopathy. This is caused by mutations in the dystrophin gene, and is related to Duchenne muscular dystrophy, in that both dystrophies result from a mutation in the dystrophin gene, but Becker has a milder course, compared to Duchenne muscular dystrophy.

Distal myopathy is a group of rare genetic disorders that cause muscle damage and weakness, predominantly in the hands and/or feet. Mutation of many different genes can be causative. Distal myopathy usually appears between ages 40 and 60. But it can sometimes show up as early as the teenage years. A non-exhaustive list of the genes involved in distal myopathy is the following: TIA1 (WDM), TTN, ZASP, GNE, DYSF, MYH7, VCP (P97), CRYAB, CAV3, and MATR3.

Congenital muscular dystrophies (CMDs) are autosomal recessively-inherited muscle diseases, characterized by muscle weakness which is present at birth and the different changes on muscle biopsy that ranges from myopathic to overtly dystrophic due to the age at which the biopsy takes place. Indeed, there have been 35 genes discovered to be involved with different forms of CMD resulting from these mutations. There are different forms of CMD, often categorized by the protein changes caused by an atypical gene. A non-exhaustive list of these genes: LAMA2, COL6A1, COL6A2, COL6A3, SEPN1, POMT1, POMT2, FKRP, ISPD, CTDC2, TMEM5, POMGnT1, B3GALnT2, GMPPB, B3GnT1, SGK196, FKTN and TMEM5.

Emery-Dreifuss muscular dystrophy (EDMD) affects muscles used for movement (skeletal muscles), causing atrophy, weakness and contractures. It almost always affects the heart, causing abnormal rhythms, heart failure, or sudden cardiac death. It is rare, affecting 0.39 per 100,000 (1 per 250,000) people. EDMD is an X-linked recessive resulting of an EMD gene mutation.

Oculopharyngeal muscular dystrophy (OPMD) is a rare form of muscular dystrophy with symptoms generally starting when an individual is 40 to 50 years old. It can be autosomal dominant (most common) or autosomal recessive. The PABPN1 mutation contains a GCG trinucleotide repeat at the 5' end of the coding region, and expansion of this repeat which then leads to autosomal dominant oculopharyngeal muscular dystrophy (OPMD) disease.

Miyoshi myopathy is a type of muscular dystrophy characterized by muscle weakness and atrophy (wasting), mainly in the distal parts of the legs. The first symptoms typically begin in young adulthood (on average 20 years of age) and include weakness and atrophy of the calves (sometimes asymmetrically), leading to inability to jump, run or walk on tiptoes. Over a period of years, the weakness and atrophy typically spread to the thighs and gluteal muscles. The forearms may become mildly atrophic with decrease in grip strength. It is caused by variations in the dysferlin gene. Inheritance is autosomal recessive. Diagnosis may be confirmed by blood tests results showing an elevation of the creatine kinase (CK) and genetic testing.

Centronuclear myopathies (CNM) are a group of congenital myopathies where cell nuclei are abnormally located in the center of muscle cells instead of their normal location at the periphery. Symptoms of CNM include severe hypotonia, hypoxia-requiring breathing assistance, and scaphocephaly. Among centronuclear myopathies, the X-linked myotubular myopathy form typically presents at birth, and is thus considered a congenital myopathy. However, some centronuclear myopathies may present later in life. Congenital forms often present with neonatal low muscle tone, severe weakness, delayed developmental milestones (particularly gross motor milestones such as head control, crawling, and walking) and pulmonary complications (presumably due to weakness of the muscles responsible for respiration). Involvement of the facial muscles may cause ophthalmoplegia or ptosis. A mutation in the RYR1 gene causing CNM may also cause susceptibility to malignant hyperthermia, a potentially life-threatening reaction to anesthesia.

The invention also relates to a method for the diagnosis, the prognosis, for monitoring the evolution, or for determining the risk of having or for developing a muscular dystrophy in a subject, or for determining the efficacy of a treatment of a muscular dystrophy in a subject, comprising detecting the presence or absence of galectin-3 or galectin-3 mRNA upregulation.

The term "upregulation" as used herein refers to an increased level of one or more galectins. According to the present invention, the term "upregulation" refers to an increased level of galectin-3 and/or galectin-3 mRNA.

The invention aims in particular at detecting the presence or absence of galectin-3 or galectin-3 mRNA upregulation in a biological fluid sample or a muscle sample of a subject, more preferably a muscle sample.

In one embodiment, the presence or absence of galectin-3 upregulation is detected by an immunoassay that is preferably selected from sandwich assay, competitive assay, western blotting or ELISA, by mass spectrometry, or by galectin puncta assay, more preferably galectin puncta assay.

The term "immunoassay" as used herein refers to a biochemical test that measures the presence or concentration of a macromolecule or a small molecule in a solution through the use of an antibody (usually) or an antigen (sometimes). The molecule detected by the immunoassay is often referred to as an "analyte" and is in many cases a protein, although it may be other kinds of molecules, of different sizes and types, as long as the proper antibodies that have the required properties for the assay are developed. Immunoassays rely on the ability of an antibody to recognize and bind a specific macromolecule in what might be a complex mixture of macromolecules. In the present invention, the detection of galectin-3 in a biological fluid sample may be done by an immunoassay. In a particular embodiment, said immunoassay may be selected from the group consisting of a sandwich assay, a competitive assay or an ELISA.

The term "competitive assay" as used herein refers to a competitive binding immunoassay that typically measures the binding of a labeled ligand to a target protein in the presence of a second, competing but unlabeled ligand. This assay can be used to assess qualitative binding information as well as relative affinities of two or more molecules for one target. There are two types of competitive assay: competitive homogeneous immunoassays and competitive heterogeneous immunoassays. In a competitive, homogeneous immunoassay, unlabeled analyte in a sample competes with labeled analyte to bind an antibody. The amount of labelled, unbound analyte is then measured. In theory, the more analyte in the sample, the more labelled analyte gets displaced and then measured; hence, the amount of labelled, unbound analyte is proportional to the amount of analyte in the sample. In a competitive heterogeneous immunoassay, as in a competitive, homogeneous immunoassay, unlabeled analyte in a sample competes with labelled analyte to bind an antibody. However, in the heterogeneous assays, the labelled, unbound analyte is separated or washed away, and the remaining labelled, bound analyte is measured.

The term "western blotting" as used herein refers to an analytical technique used in molecular biology and immunogenetics as a general method to identify the presence of a specific single protein within a complex mixture of proteins. A semi-quantitative estimation of a protein can be derived from the size and color intensity of a protein band on the blot membrane. In addition, applying a dilution series of a purified protein of known concentrations can be used to allow a more precise estimate of protein concentration. The western blot is routinely used for verification of protein production after cloning. It is also used in medical diagnostics. In addition to the application of western blot in scientific research, it is also utilized in clinical research areas. Since it can be applied to the direct protein identification process, western blot is regarded as a powerful diagnostic tool that is frequently used in the clinic setting. WB and protein detection techniques can be used to find disease biomarkers like specific proteins or antibodies. It is thought to be a viable method for identifying particular proteins during the diagnosis of diseases. The western blot method is composed of a gel electrophoresis to separate native proteins by 3-D structure or denatured proteins by the length of the polypeptide, followed by an electrophoretic transfer onto a membrane (mostly PVDF or nitrocellulose) and an immunostaining procedure to visualize a certain protein on the blot membrane. SDS-PAGE is generally used for the denaturing electrophoretic separation of proteins. SDS is generally used as a buffer (as well as in the gel) in order to give all proteins present a uniform negative charge, since proteins can be positively, negatively, or neutrally charged. This type of electrophoresis is known as SDS-PAGE (SDS-polyacrylamide gel electrophoresis). Prior to electrophoresis, protein samples are often boiled to denature the proteins present. This ensures that proteins are separated based on size and prevents proteases (enzymes that break down proteins) from degrading samples. Following electrophoretic separation, the proteins are transferred to a membrane (typically nitrocellulose or PVDF). The membrane is often then stained with Ponceau S in order to visualize the proteins on the blot and ensure a proper transfer occurred. Next the proteins are blocked with milk (or other blocking agents) to prevent non-specific antibody binding, and then stained with antibodies specific to the target protein. Lastly, the membrane will be stained with a secondary antibody that recognizes the first antibody staining, which can then be used for detection by a variety of methods. The gel electrophoresis step is included in western blot analysis to resolve the issue of the cross-reactivity of antibodies.

The term "ELISA" as used herein refers to the enzyme-linked immunosorbent assay that is commonly used for analytical biochemistry assay. ELISA is a plate-based assay technique designed for detecting and quantifying soluble substances such as peptides, proteins, antibodies, and hormones. Other names, such as enzyme immunoassay (EIA), are also used to describe the same technology. In an ELISA, the antigen (target macromolecule) is immobilized on a solid surface (microplate) and then complexed with an antibody that is linked to a reporter enzyme. Detection is accomplished by measuring the activity of the reporter enzyme via incubation with the appropriate substrate to produce a measurable product. The most crucial element of an ELISA is a highly specific antibody-antigen interaction. ELISA is a powerful method for detecting and quantifying a specific protein in a complex mixture. Although many variants of ELISA have been developed and used in different situations, they all depend on the same basic elements:
1. Coating/capture-direct or indirect immobilization of antigens to the surface of polystyrene microplate wells.
2. Plate blocking-addition of irrelevant protein or other molecule to cover all unsaturated surface-binding sites of the microplate wells.
3. Probing/detection-incubation with antigen-specific antibodies that affinity-bind to the antigens.
4. Signal measurement-detection of the signal generated via the direct or secondary tag on the specific antibody.

There are several formats used for ELISAs. These fall into either direct, indirect, or sandwich capture and detection methods. The key step is immobilization of the antigen of interest, accomplished by either direct adsorption to the assay plate or indirectly via a capture antibody that has been attached to the plate. The antigen is then detected either directly (labeled primary antibody) or indirectly (such as labeled secondary antibody).

The term "sandwich assay" as used herein refers to a sandwich ELISA, which is the most widely used ELISA assay format that indirectly immobilizes and indirectly detects the presence of the target antigen. This type of capture assay is called a "sandwich" assay because the analyte to be measured is bound between two primary antibodies, each detecting a different epitope of the antigen-the capture antibody and the detection antibody. The sandwich ELISA format is highly used because of its sensitivity and specificity.

Alternatively, the expression of a marker protein can be determined by mass spectrometry (MS) and comprises the identification of proteins by measuring the ratio between mass and charge (m/z) of peptides obtained from their enzymatic digestion (MS) or of their fragments (MS/MS). The m/z of thus generated peptides are then compared to the calculated m/z of protein sequences present in databases permitting the protein identification. According to a particular embodiment, the multiple reaction monitoring (MRM) approach can be used in the present invention, in order to allow quantitative and/or qualitative detection of the peptides in muscle samples.

In an advantageous embodiment of the present invention, the expression of galectin-3 is measured by using galectin-3 puncta assay, particularly in muscle samples.

The galectin-3 puncta assay is based on the high binding affinity of the galectin-3 protein to glycan residues (a chemical particular form of sugar). An early symptom of the lysosomal stress and damage response is a chemico-structural change in the lysosome membrane. Under normal conditions, the particular forms of the glycan residue which are recognized by Galectin-3, are masked inside the hydrophobic environment of the lysosomal membrane, in a configuration that does not permit their recognition and binding by galectin-3. Therefore, under normal conditions, Galctin-3 expression in the myofiber in a cytoplasmic diffusible pattern. However, upon lysosomal stress and membrane damage, these glycan residues are exposed to the cytoplasmic hydrophilic cellular environment in a position that favors their binding by large number of galectin-3 molecules. The biological function of galectin-3 "sensing" of the damaged lysosomal membrane, is the activation and synchronization of the lysosomal stress response (PMID: 31813797). The detection of a puncta Galectin-3 patterns, by using the anti-galectin-3 antibody, is therefore a biological biomarker for the early-stage of the lysosomal stress and damage response.

The Applicant has been able to find out that lysosomal damage seems to be a universal feature of muscular dystrophy. Since Galectin-3 is upregulated in dystrophic muscles of different models and patients and is located in the lysosomes, the Applicant developed a method of using galectin-3 puncta assay for its quantitative assessment in muscular dystrophies.

The Applicant has also been able to demonstrate that Gal-3 puncta assay can be used as a generic biomarker for the detection and the monitoring of lysosomal damage in neuromuscular diseases such as muscular dystrophies.

In another embodiment, the presence or absence of galectin-3 mRNA upregulation is detected by northern blotting, qRT-PCR or by DNA microarray.

The term "mRNA" as used herein refers to messenger ribonucleic acid (mRNA), which is a single-stranded molecule of RNA that corresponds to the genetic sequence of a gene, and is read by a ribosome in the process of synthesizing a protein. mRNA is created during the process of transcription, where an enzyme (RNA polymerase) converts the gene into primary transcript mRNA (also known as pre-mRNA). This pre-mRNA usually still contains introns, regions that will not go on to code for the final amino acid sequence. These are removed in the process of RNA splicing, leaving only exons, regions that will encode the protein. This exon sequence constitutes mature mRNA. Mature mRNA is then read by the ribosome, and, utilizing amino acids carried by transfer RNA (tRNA), the ribosome creates the protein. This process is known as translation. All of these processes form part of the central dogma of molecular biology, which describes the flow of genetic information in a biological system. As in DNA, genetic information in mRNA is contained in the sequence of nucleotides, which are arranged into codons consisting of three ribonucleotides each. Each codon codes for a specific amino acid, except the stop codons, which terminate protein synthesis. The translation of codons into amino acids requires two other types of RNA: transfer RNA, which recognizes the codon and provides the corresponding amino acid, and ribosomal RNA (rRNA), the central component of the ribosome's protein-manufacturing machinery.

The term "nucleotide(s)" as used herein generally refers to any polyribonucleotide or polydeoxyribonucleotide, which may be unmodified RNA or DNA or modified RNA or DNA. Polynucleotide(s) include, without limitation, single- and double-stranded DNA, DNA that is a mixture of single- and double-stranded regions or single- and triple-stranded regions, single- and double-stranded RNA, and RNA that is a mixture of single- and double-stranded regions, hybrid molecules comprising DNA and RNA that can be single-stranded or, more typically, double-stranded, or triple-stranded regions, or a mixture of single- and double-stranded regions. As used herein, the term "polynucleotide(s)" also includes DNAs or RNAs as described above that contain one or more modified bases. Thus, DNAs or RNAs with backbones modified for stability or for other reasons are "polynucleotide(s)" as the term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as used herein embraces such chemically, enzymatically or metabolically modified forms of polynucleotides, as well as the chemical forms of DNA and RNA characteristic of viruses and cells, including, for example, simple and complex cells. "Polynucleotide(s)" also embraces short polynucleotides often referred to as oligonucleotide(s).

The term "northern blotting" as used herein refers to northern blot or RNA blot, which is a technique used in molecular biology research to study gene expression by detection of RNA or isolated mRNA in a sample. Eukaryotic mRNA can then be isolated through the use of oligo (dT) cellulose chromatography to isolate only those RNAs with a poly(A) tail. RNA samples are then separated by gel electrophoresis. Since the gels are fragile and the probes are unable to enter the matrix, the RNA samples, now separated by size, are transferred to a nylon membrane through a capillary or vacuum blotting system. Once the RNA has been transferred to the membrane, it is immobilized through covalent linkage to the membrane by UV light or heat. After a probe has been labeled, it is hybridized to the RNA on the membrane. The membrane is washed to ensure that the probe has bound specifically and to prevent background signals from arising. The hybrid signals are then detected by X-ray film and can be quantified by densitometry.

The term "qRT-PCR" as used herein refers to a technique for performing a quantitative PCR (polymerase chain reaction) on an RNA sample. The RNA is first retrotranscribed by an enzyme called reverse transcriptase, which enables the synthesis of complementary DNA (cDNA). The cDNA is then used for quantitative PCR. Reverse transcriptase (RT) is an enzyme used by retroviruses and retrotransposons to transcribe the genetic information of viruses or retrotransposons from RNA to DNA, which can then be integrated into the host genome. qRT-PCR was developed to use RNA as the amplification template for quantitative PCR. It is certainly the most sensitive method for detecting and quantifying messenger RNA at organ, tissue or cell level.

The term "DNA microarray" as used herein refers to a DNA chip or biochip. DNA microarrays can be used to detect RNA, most commonly as cDNA after reverse transcription. In an mRNA or gene expression profiling experiment the expression levels of thousands of genes are simultaneously monitored to study the effects of certain treatments, diseases, and developmental stages on gene expression. For example, microarray-based gene expression profiling can be used to identify genes whose expression is changed in response to pathogens or other organisms by comparing gene expression in infected to that in uninfected cells or tissues.

The method according to the invention preferably comprises:
a) measuring the level of galectin-3 or galectin-3 mRNA in a sample of muscle of said subject, and
b) comparing said level to the level of said galectin-3 protein or mRNA in another muscle sample collected from said subject or from another subject, and/or to a standard galectin-3 or galectin-3 mRNA level.

The term "comparing" as used herein refers to determining if there is a significative difference between the galectin-3 or galectin-3 mRNA level detected in the muscle sample collected from the subject for whom the expression of galectin-3 is to be assessed and analogizing it to the expressions of galectin-3 in another muscle sample collected from said subject or from another subject, and/or to a standard galectin-3 or galectin-3 mRNA level.

The term "standard level" as used herein refers to a measure used as a norm in comparative evaluations.

The said sample of muscle is preferably obtained by muscle biopsy.

In an alternative embodiment of the present invention, the method preferably comprises:
a) measuring the level of galectin-3 in a sample of biological fluid of said subject,
   and
b) comparing said level to the level of said galectin-3 protein in another biological fluid sample collected from said subject or from another subject, and/or to a standard galectin-3 level.

In one embodiment, the method according to the invention is used for the prognosis or for monitoring the evolution of a muscular dystrophy in a subject in need thereof, comprising measuring the level of galectin-3 or galectin-3 mRNA in a sample of muscle of the subject, this level of galectin-3 or galectin-3 mRNA being then compared to the level of said galectin-3 or galectin-3 mRNA in a muscle sample previously collected in the same subject; the evolution of the level of said galectin-3 or galectin-3 mRNA being indicative of the progression of the disease.

The comparison between the level of galectin-3 in a given sample and the level of galectin 3 in another sample, can be carried out by any means known to the person skilled in the art. For example, comparison may be done using image analysis. The following elements may be used to compare microscopic images from samples, such as muscle samples, subjected to the galectin 3 puncta assay:
- the overall galectin-3 signal intensity is quantified,
- the galectin 3 signal distribution is analyzed and quantified, i.e. the shift from a diffused homogenous pattern into a puncta pattern, and/or
- the change of co-localization of galectin 3 with a lysosomal marker (such as in Lamp2 or any other suitable lysosomal marker) is quantified.

In an alternative embodiment, the method according to the invention is used for the prognosis or for monitoring the evolution of a muscular dystrophy in a subject in need thereof, comprising measuring the level of galectin-3 in a sample of biological fluid of the subject, this level of galectin-3 being then compared to the level of said galectin-3 in a biological fluid sample previously collected in the same subject; the evolution of the level of said galectin-3 being indicative of the progression of the disease.

In one embodiment, the method according to the invention is used for determining the efficacy of a treatment of a muscular dystrophy in a subject in need thereof, comprising measuring the level of galectin-3 or galectin-3 mRNA in a sample of muscle of the subject, this level of galectin-3 or galectin-3 mRNA being then compared to the level of said galectin-3 or galectin-3 mRNA in a muscle sample previously collected in the same subject; the evolution of the level of said galectin-3 or galectin-3 mRNA being indicative of the progression of the disease.

In an alternative embodiment, the method according to the invention is used for determining the efficacy of a treatment of a muscular dystrophy in a subject in need thereof, comprising measuring the level of galectin-3 in a sample of biological fluid of the subject, this level of galectin-3 being then compared to the level of said galectin-3 in a biological fluid sample previously collected in the same subject; the evolution of the level of said galectin-3 being indicative of the progression of the disease.

In any of the above-mentioned methods, said method may comprise:
a) measuring the level of galectin-3 in said sample of muscle previously collected in said subject, whereby a reference level is determined; then
b) measuring the level of galectin-3 in a said sample of muscle collected in the said same subject, whereby a test level is determined; and
c) comparing the reference and test levels, the evolution of the level of galectin-3 being indicative of the progression of the disease.

As used herein, the term "reference level" refers to an amount or concentration of a biomarker which may be of interest for comparative purposes. In one embodiment, a reference level may be the level of at least one biomarker expressed as an average of the level of the at least one biomarker from samples taken from a control population of healthy subjects. In another embodiment, the reference level may be the level of at least one biomarker in the same subject at an earlier time, i.e., before the present assay. In even another embodiment, the reference level may be the level of at least one biomarker in the subject prior to receiving a treatment regime.

In any of the above-mentioned methods, said method may comprise an alternative embodiment, the method according to the invention, comprises:
a) measuring the level of galectin-3 in said sample of biological fluid previously collected in said subject, whereby a reference level is determined; then
b) measuring the level of galectin-3 in a said sample of biological fluid collected in the said same subject, whereby a test level is determined; and
c) comparing the reference and test levels, the evolution of the level of galectin-3 being indicative of the progression of the disease.

The subject of the present invention is a human or non-human mammal. Non-human mammals include in particular a mouse, primate or canine subject. In a preferred embodiment, the subject is a human subject, in particular a child or a teenager. The subject may present a predisposition to a muscular dystrophy identified thanks to genome analysis, or suspected because of family history. The subject may also suffer from an already established muscular dystrophy. The method of diagnosis of the present invention may also be applied to a subject with no known symptom or predisposition. In some embodiments, the method is applied for mass screening of young children or of newborns. The invention may also be used for monitoring animal models, in particular canine models such as the Golden Retriever muscular dystrophy (GRMD) dog, or mouse models such as the mdx mouse during preclinical evaluation of treatments.

In one embodiment, the method according to the invention comprises:
a) measuring the level of galectin-3 in said sample of muscle previously collected in said subject, whereby a reference level is determined; then
b) measuring the level of galectin-3 in said sample of muscle collected in said same subject at a time after administration of said treatment, whereby a test level is determined; and
c) comparing the reference and test levels, the evolution of the level of galectin-3 being indicative of the efficacy of said treatment.

In an alternative embodiment, the method according to the invention comprises:
a) measuring the level of galectin-3 in said sample of biological fluid previously collected in said subject, whereby a reference level is determined; then
b) measuring the level of galectin-3 in said sample of biological fluid collected in said same subject at a time after administration of said treatment, whereby a test level is determined; and
c) comparing the reference and test levels, the evolution of the level of galectin-3 being indicative of the efficacy of said treatment.

According to the invention, advantageously, a reduction of the level of galectin-3 is indicative of an efficient treatment of the muscular dystrophy. Preferably, a statistically significant reduction of the level of galectin-3 is indicative of an efficient treatment of the muscular dystrophy. More preferably, the reduction of the level of galectin-3 is comprised between 5% and 100%, even more preferably between 25% and 100%, advantageously between 50% and 100% and even more advantageously between 75% and 100%.

According to the invention, the method preferably comprises the detection of the presence or absence of galectin-3 upregulation.

A further object of the present invention is the use of a kit for implementing a method according to the invention, comprising an analytical test to identify and quantify the presence or absence of galectin-3 upregulation for the quantification of lysosome damage in muscular dystrophy.

The present invention will now be described with reference to the following figures and examples.

### EXAMPLES

### Example 1: MATERIALS & METHODS

In vivo mice experiments: All animals were handled according to French and European guidelines for human care and use of experimental animals. Experiments on mice were approved by the ethical committee n°C2AE-51 of Evry and the regulatory affairs of the French Ministry of Research (MESRI) under the APAFIS number 1720. Mice were housed in a SPF barrier facility with 12-h light/dark cycle and were provided with food and water ad libitum. In this study, only male mice were used. For the in vivo studies, the operators who performed vector delivery, sample and tissue collection, and functional analyses were blinded to the treatment groups. DMD/mdx study: C57BI10 (WT/BL10) and mdx (C57BL/10ScSn-Dmdmdx/J) mice were obtained from Charles River laboratories. LGMD R5 study: the γ-sarcoglycan (Sgcg-/-) mouse model used in this study has been previously described (PMID: 9732288).

Gene therapy experiments in the mdx mouse model for Duchenne muscular dystrophy: A Full material and method chapter concerning this investigation can be found in Bourg et al, 2022 (PMID: 35216132). Briefly: The Opt-human MD1 (OH-MD1-µDYS) construct is our optimized version of the microdystrophin that was developed by the laboratories of Jeffrey Chamberlain (H2mDys) and George Dickson (MD1µDYS) and used (the dog version) in Genethons' preclinical investigation in the Golden retriever muscular dystrophy (GRMD) dog model. It is a spectrin-like repeat 4 to 23 deleted, C-terminal truncated microdystrophin (µdysΔ4-23ΔCT under the transcriptional control of the artificial Spc5.12 promoter). To obtain this version of mycrodystrophin the MD1µDYS was modified further, including additional codon-optimization (for human), removal of CpG methylation sites, removal of antisense ORFs and of sense ORFs larger than 100bp. The modified vector was designated OH-MD1-µDYS.

Generation and titration of recombinant AAV vector: HEK293-T cells, cultured in suspension, were transfected with the three plasmids coding for the adenovirus helper proteins, the AAV Rep and Cap proteins, and the ITR-flanked transgene expression cassette. Three days after the transfection, cells were harvested, chemically lysed, treated with benzonase (Merck-Millipore, Darmstadt, Germany), and filtered. Viral capsids were purified by affinity chromatography, formulated in sterile PBS, and the vector stock was stored at -80°C.

Titers of AAV vector stock were determined by using quantitative real-time polymerase chain reaction (gPCR): Viral DNA was extracted using the MagNA Pure 96 DNA and viral NA small volume kit (Roche Diagnostics, Indianapolis, IN) according to manufacturer's instructions. PCR was performed in ABI PRISM 7900 HT Sequence Detector with Absolute ROX mix (Taqman, Thermo Fisher Scientific, Waltham, MA), using the ITR-specific primers, forward 5'-CTCCATCACTAGGGGTTCCTTG-3', reverse 5'-GTAGATAAGTAGCATGGC-3' and the probe 5'-TAGTTAATGATTAACCC-3'. Mice treatment 6-weeks old mdx male mice were injected intravenously by the AAV9 OH-MD1-µDYS vector, at the titer of 3 × 1013 viral genome (vg)/kg), for the duration of 7 weeks, until sacrifice and analysis. Muscle biopsies were dissected, snap-frozen in isopentane in liquid nitrogen, and processed for RNA, protein, biochemical and histological analyses. Biopsies for histological analysis were frozen in isopentane in liquid nitrogen.

Detection of microdystrophin on transversal sections: Transverse 8µm cryosections of the TA muscle were air-dried and stored at -80°C. Slices were rehydrated 5 minutes in PBS, fixed 5 minutes in 3.7% ice-cold methanol-free PFA, permeabilized in 0.1% Triton-X100 for 5 minutes, and blocked 30 minutes in 10% goat serum in PBS (blocking buffer) and additional 30 minutes in Mouse-on-Mouse IgG 1X Blocking Solution. Sections were incubated with 1:50 primary antibody (Leica: NCL-DYSB) diluted with PBS in 10% blocking buffer overnight at 4°C. Samples were washed twice in PBS and incubated 45 minutes at room temperature (RT) with Alexa-conjugated secondary antibodies (Goat-anti-Mouse IgG1k, Thermofisher A21125), 1:1000 in 10% blocking solution at a dark humid chamber. Samples were washed three times in PBS and mounted in DAPI Fluoromount-G (Southern Biotech, ref. 0100-20). Images were digitalized using Axioscan Z1 slide scanner (Zeiss, Germany) under a Plan-Apochromat 10X/0.45 M27 dry objective (Zeiss, Germany) and using a digital CMOS camera ORCA-Flash4.0 (Hamamatsu, Japan). The size of a pixel is 0.65 µm. Tile scan images were reconstructed with ZEN software (ZEISS, Germany). Total and positive fibers were manually counted on four random images (900 × 900 pixels) per transversal section, using the ImageJ software. p-values were calculated by a one-way ANOVA. For the Immunofluorescence detection: Histological sections were cut at the center of the muscle and stained with H&E, or, by standard procedure, using the following antibody: for CD11b we used the rat monoclonal of BD Pharmingen^{®}, Réf. 550282, and for the detection of Laminin, a rabbit polyclonal antibody of Invitrogene, reference PA1-1673.

Gene therapy experiments in the mouse model for LGMD R5 v-sarcoglycanopathy: A Full material and method chapter concerning this investigation can be found in Israeli et al, 2019; (PMID: 31194043). Briefly: AAV construction and production. The human γ-SARCOGLYCAN gene under the control of the desmin promoter (AAV8-des-hgSCG) was cloned into a donor plasmid (pFastBac) by classical molecular biology technique. The recombinant baculovirus genome was then generated by transposition using the Bac-to-Bac baculovirus expression system. The resulting bacmid DNA was extracted and transfected into insect cells for production of recombinant baculovirus. Baculoviruses harbouring the Sgcg cDNA and AAV rep2/cap8 genes were used to infect spodoptera frugiperda (Sf9) insect cells for production of recombinant adeno-associated virus vector (rAAV). After 72 hours of suspension culture at 28°, the cells were collected by centrifugation and incubated in extraction buffer. Purification was performed on immuno-affinity AVB sepharose medium (GE Healthcare). Titration was performed by qRT-PCR using primers corresponding to the AAV ITR.

Mice and treatments: The g-sarcoglycan (Sgcg-/-) mouse model used in this study has been previously described (PMID: 9732288). The animals were anaesthetized with a mix of ketamine (100 mg/kg) and xylazine (10 mg/kg). The Sgcg (-/-) mice were treated by a viral vector dose of 4.5 1013 vg/kg, which was injected into the tail vein.

Detection of gamma sarcoglycan on transversal sections: Immunostaining for γ-sarcoglycan proteins was performed with a goat polyclonal anti γ-sarcoglycan (D-18) (Santa-Cruz, SC-14180). The sections were treated for 20 min with hydrogen peroxide at RT to inhibit endogenous peroxidases and blocked using the Mouse-On-Mouse kit (Vector Labs). Overnight primary antibody incubations (dilution 1/1000) were followed by 1 hour incubation with HRP-conjugated secondary goat antibody and revealed using DAB solution. Sections were mounted in Eukitt (Orsatech, France) and examined through a Leica confocal microscope (TCS SP2.AOBS microscope, Leica, Germany).

Gene therapy experiments in the HSA FKRP-Del mouse model for LGMDR9. Generation and housing of mouse models: FKRP-loxed mice were obtained as a gift from Dr Susan Brown. They had been generated by homologous recombination around exon 3 of the *Fkrp* gene. LoxP sequences were inserted around the *Fkrp* coding sequence, and the Enhanced Green Fluorescent Protein (EGFP) sequence was added after the stop codon, with an Internal Ribosome Entry Site (IRES) as promoter. A neomycine cassette was also inserted in 5' position of EGFP, flanked by Frt sequences. The cassette was further excised using the Flrp-Frt system. HSA-cre mice [B6.Cg-Tg(ACTA1-cre)79Jme/J] were provided by The Jackson Laboratory. The HSA Fkrp-Del mouse was obtained by the crossing of the FKRP-loxed with the HSA-cre mice.

Gene transfer: The 1-month-old HSA Fkrp-Del mice were treated, for the period of three months, by a viral vector dose of 5 10¹² vg/kg, which was injected into the tail vein.

Biopsy sampling and freezing: Organs and tissues were dissected out and frozen either in liquid nitrogen-cooled isopentane, and mounted for histological manipulations, either in liquid nitrogen for molecular biology use.

Vector copy number quantification: For quantification of viral genomes in muscles and organs, the homogenates were digested with proteinase K (Qiagen), then DNA was extracted from the lysates using MagNA Pure 96 apparatus (Roche), following MagNA Pure purification. Once extracted, DNA samples were quantified using a Nanodrop instrument (Thermofisher). Real-time PCR was performed on 45 ng of DNA, using LightCycler 480 real-time PCR System (Roche) with 0.25 µM of each primer and 0.125 µM of the probe according to the protocol Absolute Q-PCR ROX Mix (Thermofisher). The viral genome copy number was determined by amplification of the HBB2 polyA region of AAV genome.

Western Blotting detection of Galectin-3: Frozen sections were solubilized in the radio immunoprecipitation assay (RIPA) buffer (ThermoFisher 89901). After homogenization using the Fast Prep Bead Mill 2000 machine (6m/s for 40 seconds), all samples are centrifuged (2000 rpm, 10min/4°C). Samples are then treated with a DNAse/ Protein Inhibitor Cocktail (PIC) solution (1/1000 dilution of Benzonase^{®} Nuclease from Merck Millipore, ref.712163). Protein extracts are quantified by PIERCETM 660nm protein assay kit (Thermofisher, ref. 22662). 20 or 30µg of protein are processed for Western blotting. A mix of NuPAGE LDS sample buffer (Invitrogen NP0007) and DTT (dithiothreitol) is used for protein denaturation (10 min, 70°C). Samples are then loaded on a precast 1,5mm 4-12% Bis-TRIS gel from Invitrogen and migrated in MOPS SDS running buffer at 90V for 15min then 120V for 1h20min. Gels are then transferred to nitrocellulose stacks using the Thermo-Fisher iBlot 2 transfer system. Membranes are after that stained with LI-COR total protein staining kit (#926-11010) and scanned with a LI-COR Odyssey imaging scanner. Next, membranes are blocked with Li-COR Intercept (PBS) blocking buffer (927-70001) for 1h at RT, then incubated with a primary antibody solution (anti-galectin-3 Goat polyclonal, R&D AF1196, 1/1000 dilution) at 4°C overnight. Membranes are then washed 3 times with a Tween-Tris buffered saline (TTBS) solution (5 min, RT) and incubated with a secondary antibody solution (Donkey anti-Goat 800, LiCOR 926-32214). After, membranes are washed with TTBS (3 times, 5min) and scanned using Odyssey imaging system. Band intensities for total protein stain and secondary antibody are quantified using LI-COR image studio software (V.5.2).

Galectin-3 immunofluorescence: Slides are dried out at room temperature for 10 min. PAP-Pen is used to draw circles around muscle sections, and slides are re-hydrated in PBS for 5 minutes. Slides are then fixed in ice cold 4% paraformaldehyde (PFA) for 3 min and washed 2 times in PBS after. Slides are then permeabilized in an ice-cold methanol gradient (10 min 70%; 10 min 95%, 20 min 100%). After3 washing with PBS (5min), a solution of Goat serum (10%) is used for blocking at room temperature for 30 min. A Mouse-on-Mouse solution (Invitrogen R37621) is then used for blocking endogenous binding to mouse IgG (30 min RT). Slides are incubated then in a primary antibodies solution (LAMP2, Rat monoclonal - 1/200 abcam ab13524; LGALS3, Mouse Monoclonal IgG1 - 1/50 , SantaCruz sc32790) diluted in 1% Goat Serum. Slides are washed in TTBS solution 3 times for 5 minutes then incubated in a secondary antibody solution diluted in 1% Goat Serum for 2h at 4°C (Goat Anti-Rat Alexa488, Invitrogen A1 1006 1/200; Goat Anti-Mouse IgG1 Alexa 594 1/200). Finally, slides are washed in PBS solution 3 times for 5 minutes, rinsed in milliQ water and mounted with a DAPI-Fluoromount-G mounting media (Southern Biotech ref. 0100-20). Once slides are dried, they are imaged on a LEICA TCS-SP8 confocal microscope (Leica, Germany) using a 20X AP 0,75 objective. Images are acquired using LAS X software and treated with ImageJ for final figures.

In vitro LLOMe assay: 200K human immortalized myoblasts are plated on lbidi imaging dish with a coverslip bottom in skeletal muscle growth medium (Promocell C-23060) supplemented with 10% fetal bovine serum. Assay is done next day when cells are at 60-70% confluency, or are left to grow until confluence and then media is switched for differentiation medium (Promocell C-23061). For myotubes assay, cells are cultured for 7 days to obtain elongated multinucleated myotubes. Cells are treated with a LLOMe solution (Santacruz sc-285992) at a 2mM final concentration in the medium, for 20 min at 37°C. Cells are then washed briefly 2 times with HBSS buffer (Hanks' Balanced Salt Solution, calcium & magnesium). Afterword, cells are fixed in ice-cold 4% PFA solution for 15 minutes, washed with PBS 3 times and permeabilized for 10 minutes with 0,25% Triton. Cells are washed with PBS 3 times and then blocked with 10% GS for 40 minutes at RT. Primary antibody incubation is done overnight at 4°C (LGALS3, Mouse Monoclonal lgG1 - 1/50 in 1% GS, SantaCruz sc32790). Later, cells are wash 3 times with TTBS solution and incubated with secondary antibody solution for 2h at 4°C (Goat Anti-Mouse IgG1 Alexa 594 1/200 in 1%GS). Finally, cells are wash in PBS 3 times and DAPI-Fluormount-G is added to the dish. Dishes are imaged on a LEICA TCS-SP8 confocal microscope (Leica, Germany) using a 63× APO CS2 1.4 NA objective. Images are acquired using LAS X software and treated with ImageJ for final figures.

### Example 2: Galectin-3 is upregulated in muscular dystrophy

The purpose of this study is to develop an analytical method, for the histological detection of lysosomal damage, in transversal sections of skeletal muscles, in animal models and human patients of a range of muscular dystrophies. A puncta staining pattern of galectin-3 (Gal-3) was validated recently, in a non-muscular and non-pathological context, as a useful indicator of lysosomal stress response (Aits et al. 2015. Sensitive detection of lysosomal membrane permeabilization by lysosomal galectin puncta assay. Autophagy 11: 1408-1424.). The upregulation of Gal-3 in muscle biopsies of a mouse model of muscular dystrophy was reported previously, which however was attributed to the inflammation of the dystrophic muscle (van Putten et al. 2012). Comparison of skeletal muscle pathology and motor function of dystrophin and utrophin deficient mouse strains. Neuromuscul Disord 22: 406-417.). Thus, detection of lysosomal damage in muscle samples using Gal-3 was not previously reported. By using a Western blot analysis, the significant upregulation of Gal-3 in the Tibialis Anterior (TA) (Figure 1a and b) and the Gastrocnemius (GA) muscles (Figure 1c and d) of a 7-week-old mdx mouse was confirmed (Figure 1). Thus, in agreement with published data, this result indicates that Gal-3 is upregulated in the dystrophic muscle.

### Example 3: Galectin-3 colocalized with the lysosomal marker LAMP2 (Lysosome Associated Membrane Protein 2)

Immunofluorescence detection of galectin-3 and of the lysosomal marker LAMP2 in muscle transversal sections was done in this study. In the left column of figure 2, Lamp2 that stains specifically the lysosome, is increasingly expressed in the mdx TA muscle compared to the WT C57BI/6, which indicates lysosomal perturbation and enlargement in the mdx muscle. In the middle column, the "Puncta" pattern of LGALS3 stain, is increasingly visible in the mdx muscle compared to the muscle of the healthy control mouse. In the right column, colocalization of LGALS3 (red) and the lysosomal marker LAMP2 (green) is visible in the mdx muscle. These results revealed the upregulation of both proteins, as well as their colocalization in the dystrophic muscle in a puncta pattern. Thus, the data indicate that in the dystrophic muscle galectin-3 is expressed principally in the lysosomes (Figure 2).

### Example 4: Gal-3 expressed in the dystrophic muscle by infiltrating monocytes and inside myofibers

In inflammatory conditions Gal-3 is known to be expressed by infiltrating cells, originating from the immune system. In muscular dystrophy the dystrophic inflammatory muscle is infiltrated by such immune cells. Indeed, the interpretation in previous investigations (cited above) of the upregulation of Gal-3 in the dystrophic muscle, was that galectin-3 is expressed by infiltrating (CD11b positive) immune cells. The co-staining of muscle transversal sections with the monocyte/macrophage marker CD11b revealed indeed double positive cells for CD11b and Galectin-3. However, these double positive cells were detected in a necrotic region outside of the myofibers, whereas the majority of the Galectin-3 signal, that was detected in the myofiber cytoplasm and in a sub-sarcolemma position, was CD11b negative, excluding thus immunoinflammatory origin (figure 3). This data indicates that although Gal-3 is expressed in the dystrophic muscle by both infiltrating immune cells and inside myofibers the majority of Gal-3 stain in the dystrophic muscle is indeed contributed by the myogenic lineage.

### Example 5: Galectin-3 detects lysosomal damage in the myogenic lineage.

The lysosomotropic reagent L-leucyl-L-leucine methyl ester (LLOMe) is a bioactive molecule that accumulates in the lysosomes, where it induces a lysosomal stress response and membrane damage. A short-term (20 minutes) LLOMe treatment of human immortalized myoblasts (left) and their corresponding *in vitro* differentiated myotubes (right), resulted in the formation of Gal-3 puncta. This result validates the Gal-3 puncta assay for the detection of lysosomal damage in the myogenic lineage in both myoblasts and myotubes (myotube is an *in vitro* model of skeletal myofiber).

### Example 6: Monitoring lysosomal damage in a gene therapy model for Duchenne muscular dystrophy

The restoration of dystrophin expression in the mdx mouse model for DMD (Duchenne Muscular Dystrophy) is a useful experimental model system for gene therapy in muscular dystrophy (Bourg et al. 2022. Co-Administration of Simvastatin Does Not Potentiate the Benefit of Gene Therapy in the mdx Mouse Model for Duchenne Muscular Dystrophy. Int J Mol Sci 2022, Vol 23, Page 2016 23: 2016). However, no evaluation of lysosomal damage has yet been reported in the context of muscle gene therapy.

In this example, six-week-old male mdx mice were intravenously injected by an AAV-µ-dystrophin viral vector, for a duration of 4 weeks. Immunodetection of dystrophin expression in transversal sections of the TA muscle one month after gene therapy treatment confirmed the correct expression of the micro-dystrophin transgene and a nearly complete restoration of dystrophin expression (Figure 5a top line), and the normal architecture of the tissue was restored (Figure 5a bottom line). In agreement with the experiment which is presented in figure 2, increased expression of both Gal-3 and Lamp2, and their co-localization, were detected in the mdx muscle (figure 5b middle line). Importantly, the expression of both Gal-3 and Lamp-2 was drastically reduced in the mdx mice after gene therapy treatment (figure 5b bottom line). Of note however, in the treated mice the expression of Gal-3 was not completely normalized back to the healthy control level, suggesting residual lysosomal damage under the condition of gene therapy (Figure 5b bottom line). In addition to the *in vivo* validation of the puncta assay, this data demonstrated the possibility to use the Gal-3 as a prognostic biomarker, i.e. its capacity to provide quantitative estimation for the correction of lysosomal damage by the experimental therapy.

### Example 7: Monitoring lysosomal damage in a gene therapy model for gamma sarcoglycanopathy

The LGMD R5 muscular dystrophy is caused by mutations in the SGCG gene, coding for γ-sarcoglycan. Together with α-, β-, and δ-sarcoglycan, it participates in the composition of the sarcoglycan subcomplex of the striated myofiber. Mutation or aberrant expression of SGCG resulting in a severe muscular dystrophy. Using a mouse model, the feasibility of a gene replacement strategy in LGMD R5 was recently demonstrated (Israeli et al. 2019. An AAV-SGCG Dose-Response Study in a γ-Sarcoglycanopathy Mouse Model in the Context of Mechanical Stress. 13: 494-502.)

Histological evaluation of gene-therapy treated LGMD R5 mouse model confirm a nearly complete restoration of transgene expression (positive fibers) and tissue architecture (visible in the H&E stain) in the gene therapy treated mice (figure 6a). Of note, the antibody for the detection of the Gammasarcoglycan protein is human-specific (i. e. cannot detect the mouse gammasarcoglycan in the normal mouse and therefore both the healthy (WT) and the null (KO-PBS) mice are detected as gammasarcoglycan negative. Importantly, in similarity to the DMD gene therapy model, LGMD R5 mice exhibit elevated expression of both Lamp2 and Gal-3 and their increased puncta colocalization (figure 6b middle lane). Interestingly a nearly complete normalization of the Gal-3 expression pattern in the treated SGCG model mice has been observed (Figure 6b bottom lane). This data indicates that the Gal-3 puncta assay is not specific to Duchenne muscular dystrophy and can be used as a generic biomarker for the detection and the monitoring of lysosomal damage in several muscular dystrophies.

### Example 8: Detection of Lysosomal damage in muscular dystrophy in human biopsies

Investigations of human biopsies were conducted to confirm and validate the relevance of the Gal-3 puncta assay to several human pathologies: Pompe disease, Duchenne muscular dystrophy and LGMDR5.

Pompe disease, also known as glycogen storage disease type II (GSD2), is an autosomal recessive metabolic disorder. The disease is caused by deficiency of the lysosomal acid alpha-glucosidase enzyme, leading to severe cardiac and skeletal muscle myopathy due to progressive accumulation of lysosomal glycogen. This accumulation of excess undigested glycogen in the lysosome causes lysosomal dysfunction, which is associated with lysosomal structural deformation and membrane rupture (Thurberg et al. 2006. Characterization of pre- and post-treatment pathology after enzyme replacement therapy for Pompe disease. Lab Invest 86: 1208-1220.). Massive muscle damage patterns were observed by the Hematoxylin Eosin (H&E) staining technology, of transversal section of the young (8-month-old) human patient. Importantly, a widespread lamp2 staining confirmed the expected (typical in Pompe disease) lysosomal expansion, whereas the colocalized Gal-3 staining, was absent in the age-matched healthy control sample. This data indicated that indeed galectin-3 marks specifically the damaged lysosomal compartment in this human lysosomal pathology too (Figure 7 upper panel).

Human DMD samples were then investigated. In agreement with the mdx animal model for DMD, human DMD transversal sections also presented the puncta pattern of Gal-3 lysosomal damage (Figure 7 middle panel).

Finally, again in concordance with the animal model data, colocalization of Lamp2 and Gal-3 into enlarged damaged lysosomes were observed in muscle biopsies taken from LGMD R5 patients (Figure 7 bottom panel).

Taken together, these results confirmed conclusively the capacity to detect lysosomal damage in a variety of distinct muscular dystrophies in human patients using the Gal-3 puncta assay.

### Example 9: Monitoring lysosomal damage in a gene therapy model for LGMDR9 muscular dystrophy

LGMDR9 is caused by the lack or the drastically reduced enzymatic activity of the FKRP protein. The FKRP protein participates in the glycosylation of α-dystroglycan, which is important for the matrix anchor of skeletal myofibers. LGMDR9 is a recessive autosomal muscular dystrophy, affecting preferentially the muscles of the shoulder and pelvic girdles. The severity of the pathology is very heterogeneous. The muscular symptoms appear between the first to third decades and vary from the severe, Duchenne-like, to relatively benign courses. The heart can also be affected with sometime severe heart failure and death. The pathophysiology of LGMDR9 is yet poorly understood, and to our knowledge the participation of lysosomal damage or dysfunction have never been anticipated. Gene replacement strategy has been developed and validated in a preclinical evaluation in a mouse model (PMID: **28334834**). Based on this successful preclinical study, at present a phase 1/2 gene therapy clinical trial is promoted by GENETHON (https://www.genethon.com/first-patient-dosed-in-phase-1-2-clinical-trial-in-europe-of-gene-therapy-for-lgmd-r9).

In the present example (Figure 8), a mouse model for LGMDR9 (district from PMID: **28334834)** was created, characterized, treated by gene therapy, and muscle biopsies were subjected to the Galecin3 puncta assay for the evaluation of lysosomal damage in LGMDR9 and its correction by gene therapy.

Specifically, at the age of one month, the HSA-*Fkrp*-Del mouse (with deletion of FKRP specifically in skeletal myofibers) was treated intravenously by the AAV9 hFKRP viral vector (encoding for the human FKRP transgene) for the duration of three months, at a dose of 2.5 10¹² vg/kg.

At the age of 4 months the mice were sacrificed. Vector copy number DNA, and transgene (hFKRP) RNA expressions were detected in the dissected muscle biopsies (including the TA, the psoas, and the diaphragm) (not shown), which confirmed efficient vector transfer and transgene expression respectively. Histological correction of the dystrophic phenotype of the TA muscle was further confirmed by H&E analysis (not shown), indicating efficient correction of the dystrophic phenotype.

Increased expression of both of the lysosomal-damage biomarker Gal-3 and Lamp2 (Lamp2 green, Gal-3 red), and their co-localization, were detected in histological characterization of the TA muscle of the HSA-Fkrp del muscle (figure 8 middle line) compared to the WT. The expression of both Gal-3 and Lamp-2 was drastically reduced after gene therapy treatment (figure 8 bottom line), which indicates reduced lysosomal damage. Thus, in this gene therapy model system for LGMDR9 the Galectin-3 puncta assay detected the presence of lysosomal damage in LGMDR9 and its partial reversion by the therapeutic intervention.

## Claims

1. Use of galectin-3 or galectin-3 mRNA as a biomarker for the diagnosis, the prognosis, for monitoring the evolution, or for determining the risk of having or of developing a muscular dystrophy, or for determining the efficacy of a treatment of a muscular dystrophy.

2. Use of galectin-3 or galectin-3 mRNA according to claim 1, wherein said muscular dystrophy is Duchenne muscular dystrophy (DMD), Centronuclear myopathy related to dynamin 2 mutations, myotonic dystrophy, facioscapulohumeral muscular dystrophy, Becker muscular dystrophy (BMD), distal muscular dystrophy, congenital muscular dystrophy, Emery-Dreifuss muscular dystrophy, oculopharyngeal muscular dystrophy, limb-girdle muscular dystrophies (LGMD), or Miyoshi muscular dystrophy.

3. Use of galectin-3 or galectin-3 mRNA according to claim 2, wherein said muscular dystrophy is:
- Duchenne muscular dystrophy (DMD),
- LGMD, preferably gamma-sarcoglycanopathy (LGMD R5) or FKRP related limb girdle muscular dystrophy (LGMD R9), or
- Centronuclear myopathy related to dynamin 2 mutations.

4. A method for the diagnosis, the prognosis, for monitoring the evolution, or for determining the risk of having or for developing a muscular dystrophy in a subject, or for determining the efficacy of a treatment of a muscular dystrophy in a subject, comprising detecting, the presence or absence of galectin-3 or galectin-3 mRNA upregulation.

5. The method according to claim 4, wherein the presence or absence of galectin-3 upregulation is detected by an immunoassay that is preferably selected from sandwich assay, competitive assay, western blotting, or ELISA, by mass spectrometry, or by galectin puncta assay, more preferably galectin puncta assay.

6. The method according to claim 4, wherein the presence or absence of galectin-3 mRNA upregulation is detected by northern blotting, qRT-PCR or by DNA microarray.

7. The method according to any one of claims 4 to 6, wherein said method comprises:
a) measuring the level of galectin-3 or galectin-3 mRNA in a sample of muscle of said subject, and
b) comparing said level to the level of said galectin-3 protein or mRNA in another muscle sample collected from said subject or from another subject, and/or to a standard galectin-3 or galectin-3 mRNA level.

8. The method according to claim 4 to 6, wherein said method is for the prognosis or for monitoring the evolution of a muscular dystrophy in a subject in need thereof, comprising:
measuring the level of galectin-3 or galectin-3 mRNA in a sample of muscle of the subject, this level of galectin-3 or galectin-3 mRNA being then compared to the level of said galectin-3 or galectin-3 mRNA in a muscle sample previously collected in the same subject; the evolution of the level of said galectin-3 or galectin-3 mRNA being indicative of the progression of the disease.

9. The method according to claim 4 to 6, wherein said method is for determining the efficacy of a treatment of a muscular dystrophy in a subject in need thereof, comprising:
measuring the level of galectin-3 or galectin-3 mRNA in a sample of muscle of the subject, this level of galectin-3 or galectin-3 mRNA being then compared to the level of said galectin-3 or galectin-3 mRNA in a muscle sample previously collected in the same subject; the evolution of the level of said galectin-3 or galectin-3 mRNA being indicative of the progression of the disease.

10. The method according to claim 7, 8 or 9, comprising:
a) measuring the level of galectin-3 in said sample of muscle previously collected in said subject, whereby a reference level is determined; then
b) measuring the level of galectin-3 in a said sample of muscle collected in said same subject, whereby a test level is determined; and
c) comparing the reference and test levels, the evolution of the level of galectin-3 being indicative of the progression of the disease.

11. The method according to claim 9, comprising:
a) measuring the level of galectin-3 in said sample of muscle previously collected in said subject, whereby a reference level is determined; then
b) measuring the level of galectin-3 in said sample of muscle collected in said same subject at a time after administration of said treatment, whereby a test level is determined; and
c) comparing the reference and test levels, the evolution of the level of galectin-3 being indicative of the efficacy of said treatment.

12. The method according to anyone of claim 9 to 11, wherein a reduction of the level of galectin-3 is indicative of an efficient treatment of the muscular dystrophy.

13. The method according to anyone of claim 4 to 12, comprising the detection of the presence or absence of galectin-3 upregulation.

14. The method according to any one of claim 4 to 13, wherein the muscular dystrophy is Duchenne muscular dystrophy (DMD), Centronuclear myopathy related to dynamin 2 mutations, myotonic dystrophy, facioscapulohumeral muscular dystrophy, Becker muscular dystrophy (BMD), distal muscular dystrophy, congenital muscular dystrophy, Emery-Dreifuss muscular dystrophy, oculopharyngeal muscular dystrophy, limb-girdle muscular dystrophies (LGMD), or Miyoshi muscular dystrophy, preferably:
- Duchenne muscular dystrophy (DMD),
- LGMD, more preferably gamma-sarcoglycanopathy (LGMD R5) or FKRP related limb girdle muscular dystrophy (LGMD R9), or
- Centronuclear myopathy related to dynamin 2 mutations.

15. Use of a kit for implementing a method according to any one of claims 4 to 14, comprising an analytical test to identify and quantify the presence or absence of galectin-3 upregulation for the quantification of lysosome damage in muscular dystrophy.
